# FASCICULE DE BREVET EUROPEEN

(11) **EP 4 041 460 B1**
(45) Date de publication et mention de la délivrance du brevet: **10.12.2025**
(21) Numéro de dépôt: 20799766.9
(22) Date de dépôt: 05.10.2020
(51) Int. Cl.: B05B 11/02, B05B 11/00, B05B 11/04

(54) **DISPOSITIF DE DISTRIBUTION DE PRODUIT FLUIDE ET SON PROCEDE DE REMPLISSAGE**
VORRICHTUNG ZUM ABGEBEN EINES FLÜSSIGEN PRODUKTS UND VERFAHREN ZUM FÜLLEN DERSELBEN
DEVICE FOR DISPENSING A FLUID PRODUCT, AND METHOD FOR FILLING SAME

(30) Priorité: 07.10.2019 FR 1911085
(43) Date de publication de la demande: 17.08.2022
(73) Titulaire: APTAR France SAS, 27110 Le Neubourg (FR)
(72) Inventeur: SAVALLE, Matthieu, 76350 Oissel (FR)
(74) Mandataire: CAPRI
(86) Numéro de dépôt international: PCT/FR2020/051738
(87) Numéro de publication internationale: WO 2021/069823

(56) Documents cités:
- EP-A2- 0 925 805
- WO-A1-00/24523
- WO-A1-00/71262
- WO-A2-2004/069664
- FR-A1- 2 750 051

## Description

La présente invention concerne un dispositif de distribution de produit fluide et son procédé de remplissage.

Plus particulièrement, la présente invention concerne un dispositif de distribution de produit fluide comportant une tête de distribution de produit fluide pour distribuer une ou deux dose(s) d'un produit fluide pharmaceutique. Dans ce type de dispositif, généralement appelé unidose ou bidose, chaque dose de produit fluide, généralement liquide, est distribuée ou pulvérisée à travers un orifice de distribution lors d'un actionnement manuel de ce dispositif. Si le dispositif est un unidose, la totalité du produit fluide est distribuée en un seul actionnement. Si le dispositif est un bidose, le produit fluide est distribuée en deux actionnements successifs du dispositif.

Généralement, ce type de dispositif unidose ou bidose est utilisé pour une distribution nasale, buccale ou sublinguale du produit fluide. Dans le cas d'une distribution nasale, la tête de distribution comporte alors une extension axiale adaptée à pénétrer dans une narine d'un utilisateur lors de l'utilisation.

Ce type de dispositif peut présenter certains inconvénients. Ainsi, les dispositifs du type unidose ou bidose utilisent généralement une aiguille creuse, généralement métallique, pour percer le bouchon lors de l'actionnement. Or, l'utilisation d'une pièce métallique est couteuse, et rend le recyclage du dispositif après utilisation plus complexe. De plus, lors du perçage du bouchon par l'aiguille, des particules d'élastomère peuvent se détacher du bouchon et polluer le fluide distribué.

Les documents WO0024523, WO0071262, WO2004069664 et FR2750051 décrivent des dispositifs de l'état de la technique.

La présente invention a pour but de fournir un dispositif de distribution de produit fluide qui ne reproduit pas les inconvénients susmentionnés.

Plus particulièrement, la présente invention a pour but de fournir un dispositif qui limite le risque de contamination du produit fluide avant et pendant la distribution.

La présente invention a également pour but de fournir un tel dispositif qui soit simple et peu coûteux à fabriquer et à assembler.

La présente invention a donc pour objet un dispositif de distribution de produit fluide, comportant un corps recevant un réservoir contenant une ou deux doses d'un produit fluide, ledit réservoir comportant un bouchon qui obture ledit réservoir de manière étanche avant actionnement, ledit corps comportant une tête de distribution pourvue d'un orifice de distribution, et des moyens de distribution pour distribuer au moins une partie dudit produit fluide à travers ledit orifice de distribution lors d'un actionnement, ledit bouchon étant symétrique et réversible et donc utilisable dans le réservoir indifféremment dans un sens ou dans l'autre, ledit bouchon comportant une partie interne élastiquement déformable qui, lorsqu'une pression est appliquée d'un côté axial du bouchon ou de l'autre, se déforme pour ouvrir un passage, et qui, lorsque ladite pression cesse, revient élastiquement dans sa position de repos dans laquelle elle obture ledit passage.

Avantageusement, ladite partie interne comporte deux parties bombées radialement opposées sollicitées au repos radialement l'une contre l'autre pour fermer de manière étanche ledit passage.

Avantageusement, ledit bouchon comporte un évidement dans chaque partie bombée, pour faciliter la déformation élastique desdites parties bombées.

Avantageusement, ledit bouchon comporte une partie externe d'étanchéité, formée sur sa périphérie externe.

Avantageusement, ladite partie externe d'étanchéité comporte au moins un bourrelet périphérique réalisant l'étanchéité contre une paroi latérale interne dudit réservoir.

La présente invention a aussi pour objet un procédé de remplissage d'un dispositif de distribution de produit fluide comportant un corps, un réservoir contenant une ou deux doses d'un produit fluide, ledit réservoir comportant un bouchon qui obture ledit réservoir de manière étanche avant actionnement, une tête de distribution pourvue d'un orifice de distribution, et des moyens de distribution pour distribuer au moins une partie dudit produit fluide à travers ledit orifice de distribution lors d'un actionnement, ledit bouchon étant symétrique et réversible, ledit bouchon comportant une partie interne élastiquement déformable qui, lorsqu'une pression est appliquée d'un côté axial du bouchon, se déforme pour ouvrir un passage, et qui, lorsque ladite pression cesse, revient élastiquement dans sa position de repos dans laquelle elle obture ledit passage, ledit procédé comprenant les étapes suivantes :
- ledit bouchon est inséré dans le fond dudit réservoir,
- un tube de remplissage est inséré à travers ladite partie interne élastiquement déformable du bouchon,
- ledit réservoir est rempli avec ledit produit fluide, ledit bouchon remontant dans ledit réservoir sous l'effet de la pression hydraulique du produit fluide,
- ledit tube de remplissage est retiré après remplissage, ladite partie interne se déformant élastiquement pour refermer ledit passage.

Avantageusement, le remplissage se poursuit au début du retrait dudit tube de remplissage.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution nasale de produit fluide selon un mode de réalisation avantageux,
La figure 2 est une vue schématique de détail d'une partie du dispositif de la figure 1, avant actionnement,
La figure 3 est une vue similaire à celle de la figure 2, en cours d'actionnement,
Les figures 4 à 7 sont des vues schématiques montrant les phases de remplissage du réservoir des figures 1 à 3, et
La figure 8 est une vue schématique en section transversale d'un bouchon selon une variante de réalisation avantageuse.

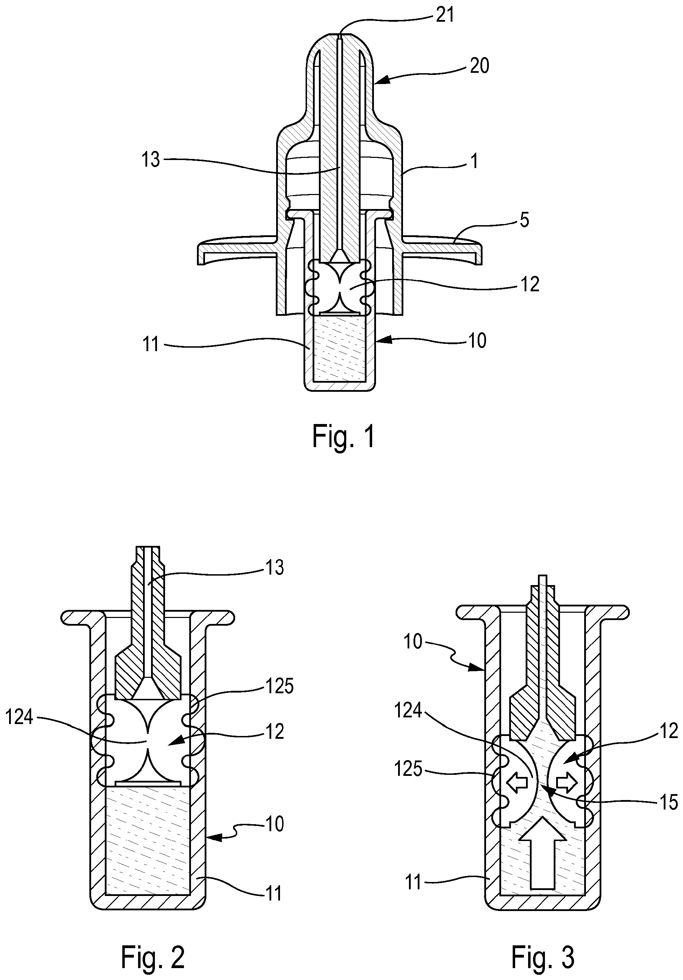

Les termes "proximal" et "distal" se réfèrent à l'orifice de distribution. Les termes "axial" et "radial" se réfèrent à l'axe central longitudinal du dispositif. Les termes "supérieur", "inférieur", "haut" et "bas" se réfèrent à la position droite du dispositif représentée sur les figures 1 à 7.

En référence à la figure 1, il est représenté un exemple d'un dispositif de distribution nasale de produit fluide auquel la présente invention s'applique. Dans cet exemple, il s'agit d'un dispositif unidose, dans lequel la totalité de la dose de produit fluide est distribuée en un seul actionnement. Il est à noter que la présente invention pourrait aussi s'adapter à d'autres types de dispositifs, tels que par exemple des dispositifs du type bidose, contenant deux doses de produit fluide à distribuer en deux actionnements successifs du dispositif.

Le dispositif comporte un corps 1, comportant une bride radiale 5 pour l'appui des doigts de l'utilisateur lors de l'actionnement.

Une tête de distribution 20, ici du type nasal, s'étend axialement à partir de ladite bride radiale 5 et comporte un orifice de distribution 21, orienté axialement. Cet orifice de distribution 21 sert à distribuer une dose de produit fluide hors de ladite tête de distribution 20 lors de l'actionnement du dispositif par un utilisateur, cette distribution étant réalisée par des moyens de distribution. Avantageusement, la tête de distribution nasale 20 est réalisée d'une seule pièce monobloc avec ledit corps 1. Il est à noter que la présente invention pourrait aussi s'appliquer à un dispositif comportant une tête de distribution buccale. Plus généralement, la présente invention s'applique à tous types de dispositifs unidose ou bidose.

Le corps 1 reçoit un réservoir 10. Typiquement, comme visible sur les figures, le réservoir 10 peut être formé par un corps creux et borgne 11, comportant une seule ouverture fermée par un bouchon 12 et contenant une seule dose de produit fluide à distribuer un actionnement unique du dispositif. Bien entendu, dans le cadre d'un dispositif bidose, le réservoir 10 pourrait contenir deux doses de produit fluide à distribuer en deux actionnements successifs.

Le bouchon 12 est réalisé en un matériau déformable, tel qu'un élastomère.

La tête de distribution 20 comporte des moyens de passage 13 qui relient, en cours d'actionnement, le réservoir 10 à l'orifice de distribution 21. Ces moyens de passage comportent avantageusement une tige creuse, de préférence monobloc avec le corps 1, mais qui pourrait aussi être réalisée séparément et assemblée dans ledit corps 1. Cette tige creuse 13 débouche sur son côté proximal dans l'orifice de distribution 21, et coopère sur son côté distal avec le bouchon 12 pour le déplacer par rapport au réservoir 10 lors de l'actionnement.

Le bouchon 12 comporte une partie externe d'étanchéité 125, formée sur sa périphérie externe. Dans l'exemple des figures, cette partie d'étanchéité 125 comporte trois bourrelets périphériques, un bourrelet supérieur, un bourrelet central et un bourrelet inférieur, qui réalisent l'étanchéité contre la paroi latérale interne du réservoir 10. Bien entendu, un nombre quelconque de bourrelets pourrait être prévu, par exemple un seul bourrelet.

Selon l'invention, le bouchon 12 comporte une partie interne 124 élastiquement déformable qui, lorsqu'une pression est appliquée d'un côté axial du bouchon 12, se déforme pour ouvrir un passage 15, comme visible sur les figures 3, 5, 6 et 8. Lorsque la pression cesse, la partie interne revient élastiquement dans sa position de repos dans laquelle elle obture ledit passage 15. Ceci permet de garantir l'intégrité du produit fluide avant l'actionnement, et aussi dans le cas d'un bidose, da garantir l'intégrité du produit fluide entre les deux doses.

Avantageusement, ladite partie interne 124 peut comporter deux parties bombées 1241, 1242 radialement opposées sollicitées au repos radialement l'une contre l'autre pour fermer de manière étanche ledit passage 15. L'application d'une pression axiale déforme radialement ces deux parties bombées en éloignement l'une de l'autre, pour ouvrir le passage. Lorsque la pression cesse, les deux parties bombées reviennent élastiquement dans leur position de repos obturante.

Avantageusement, le bouchon 12 comporte un évidement 126 dans chaque partie bombée 1241, 1242, pour faciliter la déformation élastique des parties bombées. Un tel bouchon peut notamment être réalisé en impression additive.

Comme visible sur les figures, le bouchon 12 est parfaitement symétrique et donc utilisable indifféremment dans un sens ou dans l'autre. Ce caractère réversible du bouchon 12 est avantageux, notamment lors de l'assemblage. En effet, il n'est pas nécessaire d'orienter le bouchon 12 dans un sens particulier, de sorte que l'assemblage du bouchon est simplifié et donc moins coûteux.

Lors de l'actionnement, le réservoir 10 est déplacé axialement vers le haut par rapport au corps 1. Lors de ce déplacement, le bouchon 12 s'ouvre sous la pression du produit fluide qui est incompressible, pour ouvrir ledit passage 15, puis le bouchon 12 coulisse dans le réservoir 10 pour expulser le produit à travers ladite tige creuse 13. Dans ce mode de réalisation, c'est le bouchon 12 qui forme les moyens de distribution.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier bouchon selon l'invention et du côté distal par un second bouchon standard, le volume défini entre lesdits deux bouchons contenant le produit fluide à distribuer. Lorsque l'utilisateur actionne le dispositif, il va comme décrit précédemment appuyer axialement sur le corps d'actionnement pour le faire coulisser axialement vers l'orifice de distribution. Ceci provoque le déplacement du second bouchon à l'intérieur du réservoir. Or, le produit fluide étant incompressible, ce déplacement du second bouchon va donc ouvrir le premier bouchon et le contenu du réservoir va être distribué à travers ledit premier bouchon et ladite tige creuse par le second bouchon, qui agit alors en tant que piston. Dans ce mode de réalisation, c'est le second bouchon qui forme les moyens de distribution.

Comme évoqué précédemment, l'invention pourrait aussi s'appliquer à un dispositif du type bidose. Dans ce cas, le contenu du réservoir serait distribué en deux actionnements successifs. Le document WO2014147329 décrit un exemple de dispositif bidose.

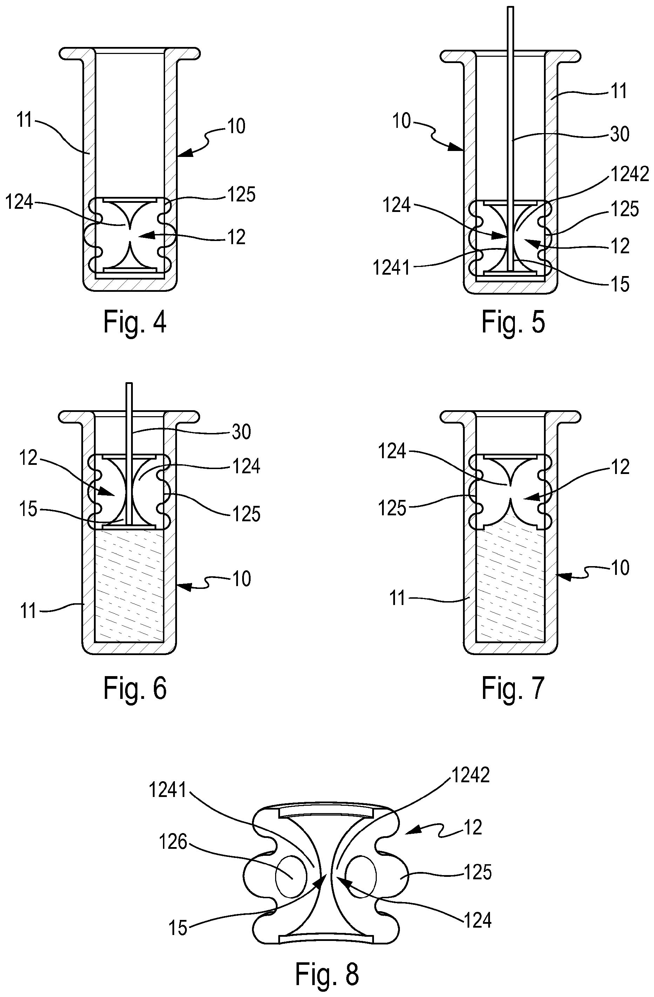

Les figures 4 à 7 montrent les étapes successives d'un procédé de remplissage avantageux.

Le bouchon 12 est d'abord inséré dans le fond du réservoir 10, comme visible sur la figure 4.

Un tube de remplissage 30 est alors inséré entre les deux parties bombées 1241, 1242, comme visible sur la figure 5, et le réservoir 10 est rempli avec ledit produit fluide, comme illustré sur la figure 6. Sous l'effet de la pression hydraulique du produit fluide, le bouchon 12 remonte dans le réservoir 10.

Le volume du réservoir disposé derrière le bouchon 12 peut donc être rempli quasi totalement, à l'exception du petit volume occupé par l'extrémité du tube de remplissage 30. Avantageusement, le remplissage se poursuit au début du retrait du tube de remplissage, jusqu'à ce que le tube atteigne le point de contact des deux parties bombées, ce qui permet de supprimer le petit volume mort lié au tube.

Lorsque le tube de remplissage 30 est complètement retiré après remplissage, les deux parties bombées 1241, 1242 se déforme élastiquement pour refermer le passage 15.

Avantageusement, cette fermeture du passage 15 chasse tout air résiduel susceptible de rester à cet endroit.

La présente invention fournit plusieurs avantages, notamment :
- elle améliore la reproductibilité du spray, en réduisant les variations d'un réservoir à l'autre ;
- elle évite l'utilisation d'une aiguille, supprimant le métal du dispositif et évitant les particules d'élastomère dans le fluide distribué ;
- elle permet d'éviter ou pour le moins de fortement limiter l'oxydation du produit fluide au contact de l'air;
- elle utilise un bouchon symétrique et réversible, ce qui simplifie sa conception et son assemblage.

Bien entendu, d'autres variantes de réalisation sont également envisageables, sans sortir du cadre de la présente invention, tel que défini par les revendications annexées.

## Revendications

1. Dispositif de distribution de produit fluide, comportant un corps (1) recevant un réservoir (10) contenant une ou deux doses d'un produit fluide, ledit réservoir (10) comportant un bouchon (12) qui obture ledit réservoir (10) de manière étanche avant actionnement, ledit corps comportant une tête de distribution (20) pourvue d'un orifice de distribution (21), et des moyens de distribution pour distribuer au moins une partie dudit produit fluide à travers ledit orifice de distribution (21) lors d'un actionnement, **caractérisé en ce que** ledit bouchon (12) est symétrique et réversible et donc utilisable dans le réservoir indifféremment dans un sens ou dans l'autre, ledit bouchon (12) comportant une partie interne (124) élastiquement déformable qui, lorsqu'une pression est appliquée d'un côté axial du bouchon (12) ou de l'autre, se déforme pour ouvrir un passage (15), et qui, lorsque ladite pression cesse, revient élastiquement dans sa position de repos dans laquelle elle obture ledit passage (15).

2. Dispositif selon la revendication 1, dans lequel ladite partie interne (124) comporte deux parties bombées (1241, 1242) radialement opposées sollicitées au repos radialement l'une contre l'autre pour fermer de manière étanche ledit passage (15).

3. Dispositif selon la revendication 2, dans lequel ledit bouchon (12) comporte un évidement (126) dans chaque partie bombée (1241, 1242), pour faciliter la déformation élastique desdites parties bombées.

4. Dispositif selon l'une quelconque des revendications précédentes, dans lequel ledit bouchon (12) comporte une partie externe d'étanchéité (125), formée sur sa périphérie externe.

5. Dispositif selon la revendication 4, dans lequel ladite partie externe d'étanchéité (125) comporte au moins un bourrelet périphérique réalisant l'étanchéité contre une paroi latérale interne dudit réservoir (10).

6. Procédé de remplissage d'un dispositif de distribution de produit fluide comportant un corps (1), un réservoir (10) contenant une ou deux doses d'un produit fluide, ledit réservoir (10) comportant un bouchon (12) qui obture ledit réservoir (10) de manière étanche avant actionnement, une tête de distribution (20) pourvue d'un orifice de distribution (21), et des moyens de distribution pour distribuer au moins une partie dudit produit fluide à travers ledit orifice de distribution (21) lors d'un actionnement, ledit bouchon (12) étant symétrique et réversible, ledit bouchon (12) comportant une partie interne (124) élastiquement déformable qui, lorsqu'une pression est appliquée d'un côté axial du bouchon (12), se déforme pour ouvrir un passage (15), et qui, lorsque ladite pression cesse, revient élastiquement dans sa position de repos dans laquelle elle obture ledit passage (15), **caractérisé en ce que** ledit procédé comprend les étapes suivantes :
- ledit bouchon (12) est inséré dans le fond dudit réservoir (10),
- un tube de remplissage (30) est inséré à travers ladite partie interne (124) élastiquement déformable du bouchon (12),
- ledit réservoir (10) est rempli avec ledit produit fluide, ledit bouchon (12) remontant dans ledit réservoir (10) sous l'effet de la pression hydraulique du produit fluide,
- ledit tube de remplissage (30) est retiré après remplissage, ladite partie interne (124) se déformant élastiquement pour refermer ledit passage (15).

7. Procédé selon la revendication 6, dans lequel le remplissage se poursuit au début du retrait dudit tube de remplissage (30).

## Patentansprüche

1. Vorrichtung zum Ausgeben eines Fluidprodukts, umfassend einen Körper (1), der ein Reservoir (10) aufnimmt, das eine oder zwei Dosen eines Fluidprodukts enthält, wobei das Reservoir (10) einen Verschluss (12) aufweist, der das Reservoir (10) vor der Betätigung abdichtend verschließt, wobei der Körper einen Ausgabekopf (20) mit einer Ausgabeöffnung (21) und Ausgabemittel aufweist, um bei einer Betätigung zumindest einen Teil des Fluidprodukts durch die Ausgabeöffnung (21) auszugeben,
**dadurch gekennzeichnet, dass** der Verschluss (12) symmetrisch und reversibel ausgeführt ist und daher im Reservoir beliebig in der einen oder anderen Richtung verwendet werden kann, wobei der Verschluss (12) einen elastisch verformbaren inneren Abschnitt (124) aufweist, der sich bei Aufbringen einer Druckkraft auf die eine oder andere axiale Seite des Verschlusses (12) verformt, um einen Durchgang (15) zu öffnen, und der bei Ausbleiben der Druckkraft elastisch in seine Ruhestellung zurückkehrt, in der er den Durchgang (15) verschließt.

2. Vorrichtung nach Anspruch 1, wobei der innere Abschnitt (124) zwei radial gegenüberliegende gewölbte Abschnitte (1241, 1242) aufweist, die in Ruhestellung radial gegeneinandergedrückt werden, um den Durchgang (15) abdichtend zu verschließen.

3. Vorrichtung nach Anspruch 2, wobei der Verschluss (12) in jedem gewölbten Abschnitt (1241, 1242) eine Aussparung (126) aufweist, um die elastische Verformung der gewölbten Abschnitte zu erleichtern.

4. Vorrichtung nach einem der vorstehenden Ansprüche, wobei der Verschluss (12) einen äußeren Dichtungsabschnitt (125) aufweist, der an seinem Außenumfang ausgebildet ist.

5. Vorrichtung nach Anspruch 4, wobei der äußere Dichtungsabschnitt (125) zumindest einen Umfangswulst aufweist, der die Abdichtung gegen eine Innenseitenwand des Reservoirs (10) bewirkt.

6. Verfahren zum Befüllen einer Vorrichtung zum Ausgeben eines Fluidprodukts, umfassend einen Körper (1), ein Reservoir (10), das eine oder zwei Dosen eines Fluidprodukts enthält, wobei das Reservoir (10) einen Verschluss (12) aufweist, der das Reservoir (10) vor der Betätigung abdichtend verschließt, einen Ausgabekopf (20) mit einer Ausgabeöffnung (21) und Ausgabemittel zum Ausgeben zumindest eines Teils des Fluidprodukts durch die Ausgabeöffnung (21) bei einer Betätigung, wobei der Verschluss (12) symmetrisch und reversibel ausgeführt ist, wobei der Verschluss (12) einen elastisch verformbaren inneren Abschnitt (124) aufweist, der sich bei Aufbringen einer Druckkraft auf eine axiale Seite des Verschlusses (12) verformt, um einen Durchgang (15) zu öffnen, und der bei Ausbleiben der Druckkraft elastisch in seine Ruhestellung zurückkehrt, in der er den Durchgang (15) verschließt,
**dadurch gekennzeichnet, dass** das Verfahren die folgenden Schritte umfasst:
- der Verschluss (12) wird am Boden des Reservoirs (10) eingesetzt,
- ein Füllrohr (30) wird durch den elastisch verformbaren inneren Abschnitt (124) des Verschlusses (12) hindurch eingeführt,
- das Reservoir (10) wird mit dem Fluidprodukt befüllt, wobei der Verschluss (12) unter der Wirkung des hydraulischen Drucks des Fluidprodukts im Reservoir (10) hochsteigt,
- das Füllrohr (30) wird nach dem Befüllen entfernt, wobei sich der innere Abschnitt (124) elastisch verformt, um den Durchgang (15) wieder zu verschließen.

7. Verfahren nach Anspruch 6, wobei das Befüllen bei beginnendem Entfernen des Füllrohrs (30) fortgesetzt wird.

## Claims

1. Device for dispensing fluid product, including a body (1) receiving a reservoir (10) containing one or two doses of a fluid product, said reservoir (10) including a plug (12) which closes off said reservoir (10) in a sealed manner before actuation, said body comprising a dispensing head (20) provided with a dispensing orifice (21), and dispensing means to dispense at least some of said fluid product through said dispensing orifice (21) during actuation, **characterised in that** said plug (12) is symmetrical and reversible and can therefore be used in the reservoir equally well in one direction or the other, said plug (12) including an elastically deformable internal part (124) which, when pressure is applied on an axial side of the plug (12) or on the other, deforms to open a passage (15), and which, when said pressure ceases, returns elastically into its rest position, in which it closes off said passage (15).

2. Device according to claim 1, wherein said internal part (124) includes two radially opposite convex parts (1241, 1242) biased at rest radially against each other to close said passage (15) in a sealed manner.

3. Device according to claim 2, wherein said plug (12) includes a recess (126) in each convex part (1241, 1242), to facilitate the elastic deformation of said convex parts.

4. Device according to any one of the preceding claims, wherein said plug (12) includes an external sealing part (125), formed on its external periphery.

5. Device according to claim 4, wherein said external sealing part (125) includes at least one peripheral bead providing sealing against an internal side wall of said reservoir (10).

6. Method for filling a device for dispensing fluid product including a body (1), a reservoir (10) containing one or two doses of a fluid product, said reservoir (10) including a plug (12) which closes off said reservoir (10) in a sealed manner before actuation, a dispensing head (20) provided with a dispensing orifice (21), and dispensing means to dispense at least some of said fluid product through said dispensing orifice (21) during actuation, said plug (12) being symmetrical and reversible, said plug (12) including an elastically deformable internal part (124) which, when pressure is applied on an axial side of the plug (12), deforms to open a passage (15), and which, when said pressure ceases, returns elastically into its rest position in which it closes off said passage (15),
**characterised in that** said method comprises the following steps:
- said plug (12) is inserted into the bottom of said reservoir (10),
- a filling tube (30) is inserted through said elastically deformable internal part (124) of the plug (12),
- said reservoir (10) is filled with said fluid product, said plug (12) rising up in said reservoir (10) under the effect of the hydraulic pressure of the fluid product,
- said filling tube (30) is removed after filling, said internal part (124) elastically deforming to close up said passage (15).

7. Method according to claim 6, wherein the filling continues at the start of the removal of said filling tube (30).
